(19) Europäisches Patentamt
European Patent Office
Office européen des brevets

(11) Publication number: **0 239 954 B1**

(12) **EUROPEAN PATENT SPECIFICATION**

(45) Date of publication of patent specification: **22.01.92**

(51) Int. Cl.5: **C07C 231/02**, C07C 231/00, C07D 295/182

(21) Application number: **87104606.6**

(22) Date of filing: **27.03.87**

(54) **A method of synthesizing acid amide.**

(30) Priority: **01.04.86 JP 72568/86**

(43) Date of publication of application:
**07.10.87 Bulletin 87/41**

(45) Publication of the grant of the patent:
**22.01.92 Bulletin 92/04**

(84) Designated Contracting States:
**CH DE FR GB IT LI SE**

(56) References cited:
**GB-A- 2 100 732**

**CHEMICAL ABSTRACTS, vol. 73, no. 18, 2nd November 1970, page 285, abstract no. 91865g, Columbus, Ohio, US; Z. SOKALSKI et al.: "Influence of dehydration method in preparation of some catalysts on their physicochemical properties"**

(73) Proprietor: **JAPAN TOBACCO INC.**
**2-1 Toranomon 2-chome Minato-ku**
**Tokyo 105(JP)**

(72) Inventor: **Matsushita, Hajime 301 Nihon Tabako**
**Umegaoka-Daiichi-Apartment 39-9, Umegaoka**
**Midori-ku Yokohama-shi(JP)**
Inventor: **Takahashi, Kyoko**
**1-12-19, Setagaya**
**Setagaya-ku Tokyo(JP)**
Inventor: **Shibagaki, Makoto 403 Nihon Tabako**
**Tsuchihashi-Daiichi-Apartment 5-6-1, Tsuchihashi**
**Miyamae-ku Kawasaki-shi(JP)**

(74) Representative: **Sajda, Wolf E., Dipl.-Phys. et al**
**MEISSNER, BOLTE & PARTNER Widenmayerstrasse 48 Postfach 86 06 24**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method of synthesizing acid amide, and particularly to a method of synthesizing acid amide by means of gas phase reaction or liquid phase reaction in the presence of a solid catalyst, and more concretely to a method of synthesizing acid amide by dehydration, transamidation or the acylation of amine.

Acid amide is a general term of materials involving a chemical bonding represented by the formula $R_1$-CO-$NR_2R_3$. In the field of the chemical industry, the acid amide is a useful material in, for example, organic solvents, plasticizers, stabilizers, dyes, medicines and agricultural chemicals and also a group of important intermediate substances for synthesizing the above-mentioned chemicals. Therefore, a large number of processes are already known for the synthesis of acid amide. Typical processes are listed below.

i A process of reacting carboxylic acid with amine, or reacting carboxylic acid derivatives with amine. To this end, acid halides, esters, lactones, acid anhydrides and ketenes may be cited as carboxylic derivatives.

ii A process of oxydizing a methyl group or a methylene group bonded with nitrogen atoms into a carbonyl group.

iii Hydration of nitrile (US Pat. 3,38l,034 (l968)), amidation by reduction of Schiff base (J. Org. Chem., 27,2640 (l962)), and carbonylation of amine (J. Am. Chem. Soc., 75,3l48 (l953)).

The easiest, most inexpensive and most widely acceptable processes among the above-mentioned synthesizing processes are synthesis based on dehydration reaction between carboxylic acid and amine, and acylation of amine by applying inexpensive carboxylic acid derivatives as an acylating reagent.

The above-mentioned synthesizing reaction based on dehydration consists in dehydration of salt formed from carboxylic acid and amine, a process expressed by the following formula:

$$R^1COOH + R^2R^3NH \rightleftarrows (R^1COO)^- (NH_2R^2R^3)^+ \rightleftarrows R^1CONR^2R^3 + H_2O$$

As seen from the above formula, the subject synthesizing reaction is a reversible reaction. When, therefore, water is removed by heating, the reaction equilibrium is shifted toward the generation of a product, thereby elevating a yield. In this case, however, a side reaction is apt to take place. Further, where ammonia is used for amine component, the dehydration of amide further proceeds, undesirably resulting in the generation of nitrile.

Low reaction temperature should preferably be applied for the saving of energy. Amidation reaction performed by dehydration, therefore, is carried out under the relatively mild conditions in the presence of a catalyst (Catalyst Course edited by Catalysis Society of Japan, vol. 8, p. 290, l985. Concrete catalysts applied to this end involve polyphosphoric acid (Chem. Pharm. Bull., l3,l065 (l965)), phosphoric acid (CA,82,l6507 z), alumina (CA,64,l443l e). However, the application of the above listed catalysts is accompanied by the undermentioned drawbacks.

Where polyphosphoric acid or phosphoric acid is applied, the drawback arises that a reactor is corroded, also making it necessary to apply the process of eliminating a catalyst after the completion of reaction. Moreover, the disposal of a waste liquid is accompanied by difficulties. Metal alkoxide are toxicated by byproduct water and tend to be deactivated. Since these alkoxide are liquids, a common problem occurs in the homogeneous catalysts. Namely, a complicated process undesirably has to be applied in order to separate the product from a catalyst.

When applied as a heterogeneous catalyst in vapor phase reaction, alumina is indeed useful. When, however, used in a liquid phase reaction, poisoning resulting from water becomes prominent, and its catalytic activity is not so high.

Description may now be made of the acylation of amine by means of carboxylic acid derivatives.

This process consists in deriving the aforementioned carboxylic acid derivatives, for example, acid halide from carboxylic acid, and subsequently reacting the carboxylic acid derivative with amine. This process does not generate water as by-product, thereby suppressing the undesirable reaction, for example, the deactivation of a catalyst by water. Moreover, the process offers the advantage of utilizing inexpensive and easily procurable materials such as ethyl acetate, dimethylformamide, and acetamide. However, the process is accompanied by an extra problem of requiring the process of converting the carboxylic acid into an active derivative such as acid halide. The process is further handicapped by an extremely slow reaction. Where, for example, amine such as n-octylamine, $\alpha$-methylbenzylamine or piperidine are converted into

acid amide with dimethylformamide, for instance, applied as an acylating reagent, the required acid amide can be obtained in the corresponding high yields of 9l%, 7l%, and 95%. For this object, however, the reactants must be heated and refluxed for as long as 53, l44 and 90 hours, respectively.

This invention has been accomplished in view of the above-mentioned circumstances and is intended to provide a method of synthesizing acid amide with a high yield in a vapor or liquid phase by applying an inexpensive heterogeneous catalyst which is free from corrosion of a reactor material, a complicated process of isolating the product after reaction, and deactivation by water.

To attain the above-mentioned object, this invention provides a process of synthesizing carboxylic acid amide by reacting amine with carboxylic acid in the presence of a catalyst in a liquid or vapor phase or reacting amine with carboxylic acid ester or carboxylic acid amide in the presence of a catalyst in a liquid or vapor phase, wherein the catalyst is a solid consisting of one or more partially dehydrated metal hydroxides whose metal is selected from the group consisting of zirconium, titanium and tin.

The essential characteristic of the present invention is the application of a catalyst consisting of a partially dehydrated solid of one or more metal hydroxides whose metal is selected from the group consisting of zirconium, titanium and tin. This partially dehydrated solid is obtained by heat treatment of the corresponding metal hydroxide under the condition in which the hydroxide is not sufficiently dehydrated to convert the hydroxide into a perfect oxide. For instance, when subjected to heat treatment at 500 °C under atmospheric pressure, zirconium hydroxide is completely dehydrated into zirconia (ZrO2). When, however, subjected to heat treatment at a temperature of about 300 °C, the zirconium hydroxide is partially dehydrated into a stable state. Application of heat treatment under the above-mentioned intermediate condition causes the zirconium hydroxide to indicate a weight loss of about l7% in about one hour. Later, however, the zirconium hydroxide shows little weight loss. Such a phenomenon similarly occurs with respect to a hydroxide of any other metal than zirconium.

The aforementioned catalyst consists of a hard and white solid. But the solid is amorphous. Therefore, the X-ray diffraction analysis cannot be used for structural analysis of the materials, and its exact chemical structure is not yet known. However, it may be assumed from the partial dehydration of the metal hydroxide that the catalyst has the bonding of metal-oxygen-metal resulting from dehydration condensation, and a hydroxyl group directly bonded to a metal is still retained. The subject catalyst is a stable mass, insoluble in water and organic solvent such as alcohol, thus capable of functioning as a heterogeneous catalytic substance. It is further ascertained that the subject catalyst has a low surface acidity and carries out ion exchange with various ions.

The subject catalysts can be prepared in a relatively simple and inexpensive way by converting oxides, chlorides or salts of their respective metals which are found abundantly in nature as mineral resources to hydroxides, which are in turn thermally treated in the respective predetermined conditions. This provides a remarkable advantage to the catalysts of the present invention over any existing metal alkoxide catalysts. Furthermore, it should be noted that the catalyst materials of the present invention can be crushed into particles of suitable sizes for use or, alternatively, they can be carried by appropriate carriers including alumina, active charcoal, silica gel, silica-alumina and zeolite.

The synthesis of acid amide according to the present invention can be conducted by either liquid phase reaction or vapor phase reaction. In this case, the subject acid amide can be produced by the conventional apparatus and process, except for the application of the above-described unique catalyst. For instance, the process and apparatus customarily used in eliminating water and other undesirable by-products produced as the result of the progress of reaction can also be utilized in the present invention.

When the subject acid amide is synthesized by the liquid phase dehydration reaction, it is advisable to prepare the raw material by dissolving 0.0l to l00 mmol, or preferably 0.l to l0 mmol, of amine relative to l mmol of carboxylic acid and heat the resultant solution in the presence of 0.0l to l0 g, or preferably 0.l to 3 g, of the aforesaid catalyst. If the amine and carboxylic acid applied as raw materials are of the volatile type, it may be advisable to supplement them during the reaction to make up for their probable loss. Further, any other solvent can be applied without any difficulty in order to perform reaction in a diluted solution, elevate reaction temperature or carry out azeotropic dehydration. Acylation reaction in a liquid phase may be conducted in the same manner as mentioned above. Namely, the subject acid amide can be obtained by dissolving l mmol of amine in the sufficient amount of ester or amide and heating the resultant solution in the presence of 0.0l to l0 g or preferably 0.l to 3 g of the aforementioned catalyst. In this case, the reaction may be conducted without any solvent or with a proper solvent.

When the reaction is completed, the catalyst will be recovered by means of filtration in any liquid phase reaction described above. The filtered solution will be dried and then distilled to isolate the product. The recovered catalyst may be reutilized.

The synthesis of the acid amide according to the present invention may be performed also in a vapour

phase through the undermentioned steps. First, the aforementioned catalytic substance is filled in a proper reaction tube to provide a catalytic bed. While the reaction tube is kept at a proper temperature, the raw material is continuously supplied to the catalytic bed. In this case, the raw material may be composed of a liquid consisting of a mixture of amine and carboxylic acid or a mixture of amine or ester or a mixture of amine and amide. It is possible to apply a proper carrier gas for feeding of the raw material. The outlet part of the reaction tube is fitted with a cooling device using ice or any other coolant. A gas drawn out of the reaction tube is condensed for trapping. The liquid thus trapped generally contains not only the required product but also unreacted masses, and in some cases, a by-product. It is advised to isolate the product in the manner resembling that which is applied in the case of a liquid phase reaction.

When the raw material, for example, amine, carboxylic acid and ester, has a low boiling point, the intended acid amide can be effectively synthesized by applying an autoclave. It is advised to perform the operation in substantially the same manner as practiced in the liquid phase reaction.

The present invention offers the advantages that acid amide can be synthesized with a high yield; since the catalytic material has a low acidity, by-products which cause coloring are not likely to be generated, thereby ensuring the production of high quality acid amide; the catalyst used in the present invention seldom tends to present the drawback of swelling or elution and posseses high heat resistance and high solvent resistance. Even when, therefore, continuously applied for long hours, the catalyst used in the present invention does not deteriorate in activity. This favorable property not only permits the reuse of the catalyst, but also prevents the release of industrial wastes, a fact bearing an extremely great industrial importance.

Description may now be made of the method of preparing the catalyst applied in this invention and of the examples of synthesizing the amide embodying the invention.

Example I (preparation of a catalyst)

200 g of zirconium oxychloride ($ZrOC\ell_2$ $8H_20$) was dissolved in I0 liters of de-ionized water. Aqueous solution of sodium hydroxide (IN) was slowly added with agitation to control pH to a level of 6.80. As a result, hydrated gel of zirconium hydroxide was obtained. After filtered, the hydrated gel was washed with de-ionized water. The washing was continued until no chloride ion was detected in the filtrate. The hydrated gel thus obtained was cut up in small pieces with a knife. The splinters were spread over a glass plate and dried at room temperature, obtaining 90 g of zirconium hydroxide. When thrown into de-ionized water after drying, the pieces of zirconium hydroxide were rapidly split into particles of various particle sizes. After the water was filtered out, the separated zirconium hydroxide was dried in an enameled vat at room temperature. The dried zirconium hydroxide particles were sieved and classified according to the mesh. Particles having the particle sizes between 24 to 60 meshes were collected in a drying container at a temperature of 300 °C and heated for 3 hours, thereby obtaining a partially-dehydrated solid of zirconium hydroxide. Heat treatment applied at this time resulted in a weight loss of about I7%.

Example 2 (preparation of a catalyst)

I90 g of titanium tetrachloride was added to I0 liters of deionized water, 28% aqueous ammonia was slowly added with thorough stirring to control the pH of the solution to 7.00, thereby producing a hydrated gel of titanium hydroxide. The hydrated gel was filtered by a Buchner funnel, and the filtrate was washed with de-ionized water until chloride ion was not detected. The resultant gel was cut up into small pieces with a knife. The splinters were spread over a glass plate and dried at room temperature. When thrown into deionized water, the dried mass was rapidly crushed into particles having various particle sizes. Then the particles were filtered. The separated titanium hydroxide was dried in an enameled vat at room temperature. The obtained titanium hydroxide was sieved and classified into various groups of particle size. Particles having 24 to 60 meshes were selected. The gathered mass was heated in a drying container held at a temperature of 300 °C for 3 hours, providing a solid of partially dehydrated titanium hydroxide.

Example 3 (manufacture of a catalyst)

26I g of tin tetrachloride was dripped into 4 liters of de-ionized water. Then 28% aqueous ammonia was slowly added with thorough agitation, and the pH of the solution was controlled to 7.0. The produced hydrated gel was filtered with a Buchner funnel. The filtered gel was washed with de-ionized water, until no chloride ion was detected. The produced hydrated gel was cut with a knife into small pieces. The splinters were dried on a glass plate at room temperature. When thrown into de-ionized water, the dried pieces were

rapidly crushed into particles having various sizes. After the water was filtered out, the separated tin hydroxide particles were dried in an enameled vat at room temperature. The tin hydroxide thus obtained was classified into various sizes by sieving. The particles having meshes ranging from 24 to 60 mesh were selected. The gathered mass was heated for 3 hours in a drying container kept at 300 °C, thereby providing a solid of partially dehydrated tin hydroxide.

Example 4 (synthesis of acid amide by vapor phase reaction with a solid of partially dehydrated zirconium hydroxide used as a catalyst: vapor phase reaction)

2 g of a solid of partially dehydrated zirconium hydroxide obtained in Example l was used as a catalyst and filled in a glass tube (measuring 4 mm in its inner diameter and 6 mm in its outer diameter). The whole mass was fixed in an electric furnace maintained at a temperature of 200 °C. Acid amide was synthesized through the undermentioned steps with the glass tube used as a reactor.

Acetic acid and n-butylamine, used as raw materials, were mixed in the molar ratio of l:5. The mixed raw materials were fed into the glass tube at the flow rate of l0 mℓ/hr at room temperature with nitrogen gas (l mℓ/sec) used as a carrier gas. The mixed raw materials were vaporized in the glass tube, and drawn out of the glass tube after reacting with the catalyst. The outcoming gas was liquefied by water cooling, and subjected to gas chromatographic analysis, indicating that N-butylacetamide was obtained with a l00% yield.

Substantially the same reaction as mentioned above was carried out, except that acetic acid and n-butylamine were mixed in the molar ratio of l0:l. In this case, too, N-butylacetamide was produced with a yield of l00%, proving that the mixing ratio of the raw materials exerted no harmful effect.

By way of comparison, acid amide was synthesized with alumina applied as a catalyst in place of a body of partially dehydrated zirconium hydroxide. Acetic acid and N-butylamine were mixed in the molar ratio of l:5 under the same condition as described above. Then the corresponding N-butylacetamide was obtained with a yield of 29%. When glass beads were used as a catalyst, and N-butylacetamide was synthesized under the same condition, the yield of N-butylacetamide was only 2%.

Example 5 (synthesis of acid amide with a solid of partially dehydrated zirconium hydroxide used as a catalyst: liquid phase reaction)

2 g of a solid of partially dehydrated zirconium hydroxide obtained in Example l was used as a catalyst and taken into a 50 mℓ flask fitted with a reflux condenser. l0 mmol of acetic acid and 50 mmol of n-butylamine were taken into the flask and slow reflux was performed under heated condition. The reaction liquid was sampled 2 hours and 5 hours later. The sampled portions were analyzed by gas chromatography. The yield of N-butylacetamide indicated 54% and l00% respectively, indicating no presence of any by-product.

By way of comparison, alumina was used as a catalyst in place of a body of partially dehydrated zirconium hydroxide. Acid amide was synthesized under the same condition as in Example 4. 5 hours later, the yield of N-butylacetamide was 3%. When glass beads were used as a catalyst, the yield of N-butylacetamide was 0.7%, even after 5 hours.

Later, various types of carboxylic acid and amine were reacted with each other using a solid of partially dehydrated zirconium hydroxide as a catalyst, the results being set forth in Table I below.

**Table 1** Liquid phase synthesis of acid amide from carboxylic acid and amine using a solid of partially dehydrated zirconium hydroxide as catalyst

| Carboxylic acid (mmol) | Amine (mmol) | Solvent | Product | Yield (%) (reaction time (hour)) |
|---|---|---|---|---|
| $CH_3COOH$ (10) | $CH_3(CH_2)_3NH_2$ (50) | None | $CH_3CONHC_4H_9$ | 54(2)[*2] 100 (5) |
| $CH_3COOH$ (10) | 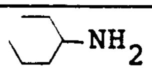 (15) | toluene | $CH_3CONH$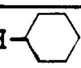 | 60 (5) |
| $CH_3COOH$ (10) | 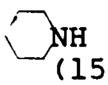 (15) | xylene | $CH_3CO$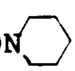 | 75 (5) |

**Note:**

(1) Reaction was carried out at the reflux temperature.

(2) Quantity of catalyst was 2 g.

Example 6 (synthesis of acid amide using a solid of partially dehydrated zirconium hydroxide as a catalyst: vapor phase reaction)

2 g of a solid of partially dehydrated zirconium hydroxide obtained in Example I was taken as a catalyst into a glass tube (measuring 4 mm in its inner diameter and 6 mm in its outer diameter). The glass tube was placed in an electric furnace kept at a temperature of 200°C. Acid amide was synthesized in the glass tube reactor through the undermentioned steps.

First, acetic acid and n-butylamine were used as raw materials. These raw materials were mixed with the molar ratio (ethyl acetate to n-butylamine) set at I0:I. The mixed raw materials were fed into the glass tube by means of a microfeeder at the rate of I0 mℓ/hr. After the mixed raw materials reacted by contact with the catalyst, gas streams drawn out of the glass tube were trapped by water cooling liquefaction. Gas chromatography showed that N-butylacetamide was produced with a I00% yield.

Thereafter, reaction was carried out between ethyl acetate and cyclohexylamine or piperidine which was respectively used in place of n-butylamine, the results being set forth in Table 2 below.

## Table 2

Vapor phase synthesis of acetic amide from ester and amine using a solid of partially dehydrated zirconium hydroxide as a catalyst

| Carboxylic ester | Amine | Product | Yield (%) |
|---|---|---|---|
| $CH_3COOC_2H_5$ | $n-C_4H_9NH_2$ | $CH_3CONHC_4H_9$ | 100 |
| $CH_3COOC_2H_5$ | 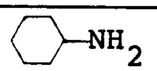 | $CH_3CONH$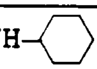 | 100 |
| $CH_3COOC_2H_5$ | 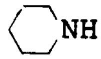 | $CH_3CO$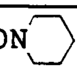 | 96 |

Example 7 (synthesis of acid amide using a solid of partially dehydrated zirconium hydroxide as a catalyst: liquid phase reaction)

2 grams of a solid of partially dehydrated zirconium hydroxide obtained in Example I were taken as a catalyst into a 50 mℓ flask fitted with a reflux condenser. 50 mmol of ethyl acetate and 5 mmol of n-butylamine were introduced into the flask and subjected to slow reflux with heat.

5 hours later, part of the reaction mixture was sampled. Gas chromatography showed that N-butylacetamide was obtained with a yield of 79%. No byproduct was observed.

Substantially the same reaction was performed as described above, except that n-butylamine was replaced by cyclohexylamine. 5 hours later, N-hexahydroacetanilide was produced with a yield of 80%. When the amine used was piperidine, N-acetylpiperidine was obtained with a yield of 60% after 5 hours.

Example 8 (synthesis of acid amide using a solid of partially dehydrated zirconium hydroxide as a catalyst: vapor phase reaction)

2 g of a solid of partially dehydrated zirconium hydroxide obtained in Example I were filled as a catalyst in a glass tube measuring 6.5 mm in its inner diameter and 8.5 mm in its outer diameter. The glass tube was placed in an electric furnace maintained at a temperature of I50 °C. Acid amide was synthesized in the glass tube reactor through the following steps.

First, a solution obtained by dissolving I0 mmol of acetic acid in 50 mℓ of benzene was taken into the glass tube by means of a microfeeder at the rate of 5 mℓ/hr. At this time, nitrogen was used as a carrier gas at the rate of 60 mℓ/sec. Then, a required quantity of ammonia gas was supplied to the tube. Gas streams obtained by the reaction of acetic acid with the ammonia gas in the presence of the above-mentioned catalyst were drawn out of the glass tube. The gas streams were trapped by water cooling liquefaction.

Gas chromatography showed that acetamide was obtained with a yield of 97%.

Reaction was performed substantially in the same manner as mentioned above, except that the reaction temperature was changed. When the reaction temperature was set at 200 °C, the main product was acetonitrile. When the reaction temperature was I00 °C, ammonium acetate was precipitated, and no desired production advanced.

When reaction was performed substantially in the same manner as mentioned above with acetic acid replaced by propionic acid, propionamide was obtained with a yield of 88% when the reaction temperature was set at I50 °C.

Example 9 (synthesis of acid amide using a solid of partially dehydrated zirconium hydroxide as a catalyst: liquid phase reaction)

2 grams of a solid of partially dehydrated zirconium hydroxide obtained in Example I were taken into a 50 mℓ flask fitted with a reflux condenser together with I0 mmol of propionic acid, and I0 mℓ of toluene. After a sufficient amount of ammonia gas was bubbled into the solution, heating was applied by means of a mantle heater, and the mixture solution in the flask was subjected to slow reflux. After 5 hours, the reaction liquid was sampled. Gas chromatography showed that propionamide was obtained with a yield of 3I%.

When reaction was carried out in the same manner as mentioned above except that ammonia gas was supplemented hourly while the reaction was continued, propionamide was obtained with a yield of 5I% after 4 hours, 68% after I0 hours and 70% after I5 hours. Where reaction took place in an autoclave kept at a temperature of I20 °C, propionamide was obtained with a yield of 87% after 5 hours.

Example I0 (synthesis of acid amide using a solid of partially dehydrated zirconium hydroxide as a catalyst: liquid phase reaction)

2 grams of a solid of partially dehydrated zirconium hydroxide were taken into a 50 mℓ flask fitted with a reflux condenser together with I0 mmol of ammonium acetate and 8 mℓ of toluene. The mixture solution received in the flask was refluxed under a heated condition, producing acetamide with a yield of 40% after 5 hours.

By way of comparison, synthesis of acetamide was tried under substantially the same conditions as mentioned above, except that zirconium hydroxide was replaced by zeolite. After 5 hours, however, no acetamide was produced.

Example II (synthesis of acid amide using a solid of partially dehydrated zirconium hydroxide as a catalyst: vapor phase reaction)

2 g of a solid of partially dehydrated zirconium hydroxide obtained in Example I were placed as a catalyst in a glass tube (measuring 6.5 mm in its inner diameter and 8.5 mm in its outer diameter). The glass tube was placed in an electric furnace kept at a temperature of 200 °C. I0 mmol of acetic acid dissolved in a 28% aqueous solution of ammonia were fed into the reaction tube by means of a microfeeder at a rate of 5 mℓ/hr. At this time, 60 mℓ/min of nitrogen gas was applied as a carrier. Gas streams drawn out of the reaction tube were trapped. Gas chromatography showed that acetamide was produced with a yield of 58%.

Synthesis of acid amide was tried under substantially the same condition as mentioned above, except that acetic acid was replaced by propionic acid, cyclohexanecarboxylic acid and pivalic acid each time. At this time the corresponding propionamide, cyclohexanecarboxamide and pivalamide were produced with yields of 53%, 57% and 52% respectively.

By way of comparison, acid amide was synthesized from propionic acid dissolved in a 28% aqueous solution of ammonia with alumina or quartz wool used as a catalyst. In this case no propionic acid amide was produced.

Example I2 (synthesis of acid amide using a solid of partially dehydrated zirconium hydroxide as a catalyst: vapor phase reaction)

2 g of a solid of partially dehydrated zirconium hydroxide obtained in Example I were placed as a catalyst in a glass tube (measuring 6.5 mm in its inner diameter and 8.5 mm in its outer diameter). The glass tube was fixed in an electric furnace kept at 200 °C. A raw material prepared by dissolving 5 mmol of acetamide and 50 mmol of cyclohexyl amine in 50 mℓ of benzene was fed into the glass tube by a microfeeder at the rate of 5 mℓ/hr. At this time, a carrier gas of nitrogen was also taken into the glass tube at the rate of 60 m /min. Gas streams drawn out of the reaction tube were trapped. Quantitative analysis based on gas chromatography showed that N-hexahydroacetanilide was produced with a yield of I00%.

Thereafter, cyclohexyl amine was replaced by piperidine, dibutylamine and n-decylamine. The reaction was carried out under the same conditions as mentioned above. The corresponding products: N-acetylpiperidine, N-acetyl-dibutylamine and N-acetyldecylamine were obtained respectively with yields of 67%, 90% and 95%.

When a mixture of I0 mmol of acetamide and 500 mmol of n-butylamine was used as a raw material, N-butylacetamide was obtained with a yield of 93%.

Example I3 (synthesis of acid amide using a solid of partially dehydrated zirconium hydroxide as a catalyst: liquid phase reaction)

2 g of a solid of partially dehydrated zirconium hydroxide were taken as a catalyst into a 50 mℓ flask fitted with a reflux condenser. 2 mmol of acetamide, 20 mmol of cyclohexylamine and 6 mℓ of toluene were received in the flask. The mixture solution received in the flask were slowly refluxed under a heated condition. After 2 hours, the reaction liquid was sampled to quantitatively analyze the produced N-hexahydroacetanilide, which was obtained with a yield of l00%.

The cyclohexylamine used in the above-mentioned process was replaced by n-butylamine, piperidine, n-decylamine, dibutylamine and n-propylamine each time. Reaction was carried out under the same conditions as mentioned above, the results being set forth in Table 3 below.

**Table 3**

Synthesis of acid amide from amides and amines using a solid of partially dehydrated zirconium hydroxide as a catalyst

| Amides | Amines | Products | Reaction time (hr) | Yield (%) |
|---|---|---|---|---|
| $CH_3CONH_2$ | 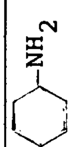 | 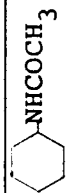 | 2 | 100 |
| $CH_3CONH_2$ | $CH_3(CH_2)_3NH_2$ | $CH_3(CH_2)_3N(H)COCH_3$ | 5 | 91 |
| $CH_3CONH_2$ | 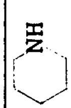 | 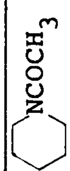 | 5 | 71 |
| $CH_3CONH_2$ | $CH_3(CH_2)_9NH_2$ | $CH_3(CH_2)_9N(H)COCH_3$ | 5 | 100 |
| $CH_3CONH_2$ | $(CH_3(CH_2)_3)_2NH$ | $(CH_3(CH_2)_3)_2NCOCH_3$ | 5 | 100 |
| $CH_3CONH_2$ | $(CH_3(CH_2)_2)_2NH$ | $(CH_3(CH_2)_3)_2NCOCH_3$ | 5 | 74 |

Example I4 (synthesis of acid amide using a solid of partially dehydrated zirconium hydroxide as a catalyst: liquid phase reaction)

2 g of a solid of partially dehydrated zirconium hydroxide obtained in Example I were added to a

mixture of 2 mmol of cyclohexylacetamide, 20 mmol of piperidine and 6 mℓ of xylene. The mixture solution was slowly refluxed. After 5 hours, the reaction liquid was sampled. Gas chromatography showed that N-acetylpiperidine was produced with a yield of 6l%.

Example l5 (synthesis of acid amide using a solid of partially dehydrated titanium hydroxide as a catalyst: vapor phase reaction)

2 g of a solid of partially dehydrated titanium hydroxide obtained in Example 2 was filled as a catalyst in a glass tube (measuring 9 mm in its inner diameter and ll mm in its outer diameter). The glass tube was fixed in an electric furnace kept at a temperature of 200 °C. A solution of acetic acid mixed with n-butylamine in the molar ratio of l:5 was fed into the glass tube by means of a microfeeder at the rate of 5 mℓ/hr. Thus acid amide was synthesized in a vapor phase. At this time, nitrogen gas was supplied as a carrier at the rate of 30 mℓ/min. As in the aforementioned vapor phase reaction, gas streams drawn out of the glass tube were trapped. Gas chromatography showed that N-butylacetamide was produced with a yield of l00%.

Example l6 (synthesis of acid amide using a solid of partially dehydrated titanium hydroxide as a catalyst: liquid phase reaction)

2 g of a solid of partially dehydrated titanium hydroxide obtained in Example 2 were taken into a 50 mℓ flask having a round bottom together with 2 mmol of acetic acid and l0 mmol of n-butylamine. The mixture solution received in the flask were slowly refluxed under heated condition. After 5 hours, the reaction liquid was sampled. Gas chromatography showed that N-butylacetamide was produced with a yield of 85%.

Example 17 (synthesis of acid amide using a solid of partially dehydrated tin hydroxide as a catalyst: vapor phase reaction)

2 g of a solid of partially dehydrated tin hydroxide obtained in Example 3 were taken as a catalyst into a glass tube (measuring 6.5 mm in inner diameter and 8.5 mm in outer diameter). The glass tube was placed in an electric furnace kept at a temperature of 200 °C. While nitrogen gas was introduced into the glass tube as a carrier at the rate of 30 mℓ/min, acetic acid and n-butyl amine were carried into the glass tube by means of a microfeeder. At this time, acetic acid and n-butylamine were mixed in the molar ratio of 1:5 and taken into the glass tube at the rate of 5 mℓ/hr. Gas streams drawn out of the glass tube were trapped. Gas chromatography showed that N-butylacetamide was produced with a yield of 70%.

Example 18 (synthesis of acid amide using a solid of partially dehydrated tin hydroxide as a catalyst: liquid phase reaction)

2 g of a solid of partially dehydrated tin hydroxide obtained in Example 3 were taken as a catalyst into a 50 mℓ flask with a round bottom together with 10 mmol of ethylacetate and 1 mmol of n-butylamine. The mixture solution received in the flask was slowly refluxed under a heated condition. After 5 hours, the reaction liquid was sampled. Gas chromatography indicated that N-butylacetamide was produced with a yield of 100%.

Example 19 (synthesis of acid amide using a solid of partially dehydrated zirconium hydroxide as a catalyst: liquid phase reaction)

2 g of a solid of partially dehydrated zirconium hydroxide obtained in Example 1 were taken as a catalyst into a 50 mℓ flask with a round bottom together with 2 mmol of piperidine and 6 mℓ of dimethylformamide. The mixture solution received in the flask was slowly refluxed under a heated condition. Reaction was brought to an end within 2 hours. N-formylpiperidine was produced with a yield of l00%.

Further, reaction was performed substantially under the same condition as described above, except that piperidine was replaced by α-methylbenzylamine, decylamine and cyclohexylamine each time. In all cases, formylation was brought to an end within 2 hours. The corresponding products (N-formyl-α-methylbenzylamine, N-formyldecylamine and N-formylcyclohexylamine) were all obtained with quantitative yields.

Example 20 (synthesis of acid amide using a solid of partially dehydrated titanium hydroxide as a catalyst: vapor phase reaction)

2 g of a solid of partially dehydrated titanium hydroxide obtained in Example 2 were filled in a glass tube (measuring 6.5 mm in its inner diameter and 8.5 mm in its outer diameter). The glass tube was placed in an electric furnace kept at a temperature of 200 °C. While nitrogen gas was supplied into the glass tube as a carrier at the rate of 30 mℓ/min, a mixed solution of ethylacetate and n-butylamine was also introduced into the glass tube by means of a microfeeder. This time, ethylacetate was mixed with n-butylamine in the molar ratio of l0:l. The mixture was introduced into the glass tube at the rate of 5 mℓ/hr. Gas streams drawn out of the glass tube were trapped. Gas chromatography showed that N-butylacetamide was produced in a quantitative yield.

Example 2l (synthesis of said acid using a solid of partially dehydrated titanium hydroxide as a catalyst: liquid phase reaction)

2 g of a solid of partially dehydrated titanium hydroxide obtained in Example 2 were taken as a catalyst into a flask with a round bottom together with 2 mmol of piperidine and 20 mmol of dimethylformamide. The mixture solution received in the flask was slowly refluxed under heated condition. After 5 hours, the reaction liquid was sampled. Gas chromatography indicated that N-formylpiperidine was produced with a yield of l00%.

Piperidine was replaced by N-butylamine, and reacted in the same manner as mentioned above. In this case N-formylbutylamine was obtained with a yield of l00%.

Example 22 (synthesis of acid amide using a solid of partially dehydrated tin hydroxide as a catalyst: vapor phase reaction)

2 g of a solid of partially dehydrated tin hydroxide obtainel in Example 3 were taken as a catalyst into a glass tube (measuring 6.5 mm in its inner diameter and 8.5 mm in its outer diameter). The glass tube was placed in an electric furnace kept at 200 °C. Nitrogen was introduced into the glass tube as a carrier at the rate of 30 mℓ/min. A solution of ethylacetate mixed with n-butylamine in the molar ratio of l0:l was fed into the glass tube by means of a microfeeder at the rate of 5 mℓ/hr. Reaction gases drawn out of the glass tube were trapped. Gas chromatography showed that N-butylacetamide was produced with a quantitative yield.

**Claims**

1. A method of synthesizing carboxylic acid amide by reacting amine with carboxylic acid in the presence of a catalyst in a liquid or vapor phase or reacting amine with carboxylic acid ester or carboxylic acid amide in the presence of a catalyst in a liquid or vapor phase, characterized in that said catalyst is a solid consisting of one or more partially dehydrated metal hydroxides whose metal is selected from the group consisting of zirconium, titanium and tin.

2. The method of synthesizing carboxylic acid amide according to claim l, characterized in that said partially dehydrated metal hydroxide is supported on a proper carrier.

3. The method of synthesizing carboxylic acid amide according to claim l or 2 characterized in that said catalyst is repeatedly used.

4. The method of synthesizing carboxylic acid amide according to any of claims l to 3, characterized in that the synthesis of carboxylic acid amide is carried out in a liquid phase, and after the reaction, the catalyst is recovered by filtration.

5. The method of synthesizing carboxylic acid amide according to claim 4, characterized in that not only a raw material but also a proper solvent is applied.

6. The method of synthesizing carboxylic acid amide according to claim 4 or 5, characterized in that the raw material has a low-boiling point, and reaction is carried out in an autoclave.

7. The method of synthesizing carboxylic acid amide according to any of the claims l to 3, characterized in that the catalyst is placed in a proper reaction tube to provide a catalyst bed; the vaporized raw material is continuously supplied to the reaction tube in contact with the catalyst bed, thereby carrying

out reaction; and outcoming reaction gases are trapped by cooling at the outlet of the reaction tube.

## Revendications

1. Procédé de synthèse d'amide d'acide carboxylique, par réaction d'une amine avec un acide carboxylique, en présence d'un catalyseur, en phase liquide ou vapeur, ou par réaction d'une amine avec un ester d'acide carboxylique ou un amide d'acide carboxylique, en présence d'un catalyseur, en phase liquide ou vapeur, caractérisé en ce que ledit catalyseur est un solide constitué d'un ou plusieurs hydroxydes de métaux partiellement déshydratés, dont le métal est choisi dans le groupe constitué par le zirconium, le titane et l'étain.

2. Procédé de synthèse d'amide d'acide carboxylique, conforme à la revendication 1, caractérisé en ce que ledit hydroxyde de métal partiellement déshydraté est supporté sur un support approprié.

3. Procédé de synthèse d'amide d'acide carboxylique, conforme à la revendication 1 ou 2, caractérisé en ce que l'on utilise le catalyseur de façon répétée.

4. Procédé de synthèse d'amide d'acide carboxylique, conforme à l'une quelconque des revendications 1 à 3, caractérisé en ce que la synthèse d'un amide d'acide carboxylique est effectuée en phase liquide, et en ce qu'après la réaction, le catalyseur est récupéré par filtration.

5. Procédé de synthèse d'amide d'acide carboxylique, conforme à la revendication 4, caractérisé en ce que l'on utilise non seulement des produits de départ, mais également un solvant approprié.

6. Procédé de synthèse d'amide d'acide carboxylique, conforme à la revendication 4 ou 5, caractérisé en ce que les produits de départ présentent un bas point d'ébullition et en ce que la réaction est effectuée dans un autoclave.

7. Procédé de synthèse d'amide d'acide carboxylique, conforme à l'une quelconque des revendications 1 à 3, caractérisé en ce que le catalyseur est placé dans un tube approprié de réaction, pour former un lit catalytique; les produits de départ vaporisés sont envoyés en continu dans le tube de réaction, au contact du lit catalytique, ce qui fait que la réaction a lieu; et les gaz issus de la réaction sont piégés par refroidissement à la sortie du tube de réaction.

## Patentansprüche

1. Verfahren zum Synthetisieren von Carbonsäureamid durch Umsetzen von Amin mit Carbonsäure in Gegenwart eines Katalysators in der flüssigen Phase oder der Dampfphase oder durch Umsetzen von Amin mit Carbonsäureester oder Carbonsäureamid in Gegenwart eines Katalysators in der flüssigen Phase oder der Dampfphase, dadurch gekennzeichnet, daß der Katalysator ein aus einem oder mehreren teilweise dehydratisierten Metallhydroxiden bestehender Feststoff ist, dessen Metall aus der aus Zirkon, Titan und Zinn bestehenden Gruppe ausgewählt ist.

2. Verfahren zum Synthetisieren von Carbonsäureamid nach Anspruch 1, dadurch gekennzeichnet, daß das teilweise dehydratisierte Metallhydroxid auf einem geeigneten Träger gehalten wird.

3. Verfahren zum Synthetisieren von Carbonsäureamid nach Anspruch 1 oder 2, dadurch gekennzeichnet, daß der Katalysator wiederholt verwendet wird.

4. Verfahren zum Synthetisieren von Carbonsäureamid nach einem der Ansprüche 1 bis 3, dadurch gekennzeichnet, daß die Synthese von Carbonsäureamid in der flüssigen Phase durchgeführt und nach der Umsetzung der Katalysator durch Filtration wiedergewonnen wird.

5. Verfahren zum Synthetisieren von Carbonsäureamid nach Anspruch 4, dadurch gekennzeichnet, daß nicht nur ein Ausgangsstoff, sondern auch ein geeignetes Lösungsmittel angewendet wird.

6. Verfahren zum Synthetisieren von Carbonsäureamid nach Anspruch 4 oder 5, dadurch gekennzeichnet, daß der Ausgangsstoff einen niedrigen Siedepunkt hat und die Umsetzung in einem Autoklaven

durchgeführt wird.

7. Verfahren zum Synthetisieren von Carbonsäureamid nach einem der Ansprüche 1 bis 3, dadurch gekennzeichnet, daß der Katalysator in ein geeignetes Reaktionsrohr gegeben wird, um ein Katalysatorbett zu schaffen; daß der verdampfte Ausgangsstoff in Kontakt mit dem Katalysatorbett kontinuierlich dem Reaktionsrohr zugeführt wird, wodurch die Umsetzung durchgeführt wird; und daß die austretenden Reaktionsgase an dem Auslaß des Reaktionsrohres durch Kühlen aufgefangen werden.